# EUROPÄISCHE PATENTSCHRIFT

(11) **EP 1 368 067 B1**
(45) Veröffentlichungstag und Bekanntmachung des Hinweises auf die Patenterteilung: **30.04.2008**
(21) Anmeldenummer: 02712934.5
(22) Anmeldetag: 02.03.2002
(51) Int. Cl.: A61L 2/20, A61L 2/18, A61L 2/16, A61L 2/07, B65B 55/10

(54) **VORRICHTUNG ZUM STERILISIEREN VON ZU BEFÜLLENDEN BEHÄLTERN**
DEVICE FOR STERILIZING CONTAINERS WHICH ARE TO BE FILLED
DISPOSITIF POUR STERILISER DES RECIPIENTS A REMPLIR

(30) Priorität: 14.03.2001 DE 10112971
(43) Veröffentlichungstag der Anmeldung: 10.12.2003
(73) Patentinhaber: PTM Packaging Tools Machinery PTE. Ltd., Singapore 049513 (SG)
(72) Erfinder: FROST, Robert, 84034 Landshut (DE); AWAKOWICZ, Peter, 81371 München (DE); STAHLECKER, Werner, 73033 Göppingen (DE)
(74) Vertreter: Patentanwälte Ruff, Wilhelm, Beier, Dauster & Partner
(86) Internationale Anmeldenummer: PCT/EP2002/002269
(87) Internationale Veröffentlichungsnummer: WO 2002/072158

(56) Entgegenhaltungen:
- WO-A-00/35495
- WO-A-98/01344
- DE-A- 1 934 363
- DE-A- 19 916 478

## Beschreibung

Die Erfindung betrifft eine Vorrichtung zum Sterilisieren von zu befüllenden Behältern, mit einer Vielzahl von mit einer kontinuierlichen oder getakteten Förderbewegung antreibbaren, jeweils einen Behälter aufnehmenden Sterilisationskammern, die in zeitlichem Abstand wenigstens zwei stationär angeordnete Sektoren durchlaufen, denen jeweils eine Unterdruckkammer unterschiedlichen Druckniveaus zugeordnet ist, an welche die Sterilisationskammern anschließbar sind. Die Erfindung betrifft auch ein Verfahren zum Sterilisieren von zu befüllenden Behältern.

Eine Vorrichtung dieser Art ist durch die DE 199 16 478 A1 Stand der Technik. Bei der bekannten Vorrichtung sind die Sterilisationskammern als Reaktoren ausgebildet, in denen jeweils ein Behälter mittels eines Niederdruckplasmas sterilisiert wird. Hierzu müssen die Sterilisationskammern vom Atmosphärendruck auf einen Plasmaentladungsdruck evakuiert werden. Die sterilisierende Wirkung eines Plasmas beruht sowohl auf einer mechanischen Zerstörung der Keime durch lonenbeschuss als auch auf einer chemischen Zerstörung durch entstehende Radikale. Wenn bei der bekannten Vorrichtung innerhalb kürzester Zeit eine Vielzahl von Behältern und Sterilisationskammern auf einen Plasmaentladungsdruck von beispielsweise 0,2 mbar zu evakuieren ist, dann sind Gasvolumina abzupumpen, die auf dem Niveau des Plasmaentladungsdruck ohne weiteres Tausende von Litern pro Sekunde erreichen können. Aus diesem Grunde sind bei der bekannten Vorrichtung wenigstens zwei aufeinander folgende, von einander getrennte Evakuierungsstufen vorgesehen, wobei der Reaktor in einer ersten Evakuierungsstufe auf einen Zwischendruck und in der zweiten Evakuierungsstufe auf den Plasmaentladungsdruck evakuiert wird.

Es hat sich nun gezeigt, dass bei praktischen Maschinenanlagen die bekannte Plasmasterilisation gewisse Probleme bereitet. Dies liegt unter anderem darin begründet, dass eine ausreichend wirksame Sterilisation vor allem nur dort stattfindet, wo das gezündete Plasma hin gelangt. Da die Behälter jedoch mit einem Transportmittel transportiert werden, gibt es zwangsläufig Behälterflächen, die von der sterilisierenden Wirkung des Plasmas ausgeschlossen sind. Außerdem liegt der Plasmaentladungsdruck auf einem Niveau, welches einen enormen technischen Aufwand erfordert und dadurch dem Wirkungsgrad abträglich ist.

Aus der internationalen Patentveröffentlichung WO 98/01344 ist eine Vorrichtung zum Sterilisieren von zu befüllenden Behältern bekannt, die eine Vielzahl von auf einem Kreisumfang angeordneten Kammern besitzt, die auf ihrer Rückseite mittels eines flexiblen Gurts verschlossen werden können. Die Behälter in diesen Kammern werden zunächst evakuiert, dann mit Wasserstoffperoxyd beaufschlagt, und daraufhin durch mehrfaches Anlegen von Unterdruck bzw. warmer, steriler Luft, wieder getrocknet.

Der Erfindung liegt die Aufgabe zu Grunde, unter Beibehaltung des eingangs genannten Konzeptes, allerdings ohne Zünden eines Plasmas, eine Vorrichtung und ein Verfahren zum Sterilisieren von zu befüllenden Behältern zu schaffen, bei welcher der Wirkungsgrad verbessert wird und ein deutlich geringerer Unterdruck erforderlich ist.

Die Aufgabe wird dadurch gelöst, dass die dem ersten Sektor zugeordnete erste Unterdruckkammer ein Druckniveau aufweist, das ein Einströmen eines Wasserdampf und Wasserstoffperoxiddampf enthaltenden Dampfgemisches in die Sterilisationskammern sowie ein Kondensieren des Dampfgemisches an den Oberflächen der zu sterilisierenden Behälter ermöglicht, dass die dem zweiten Sektor zugeordnete zweite Unterdruckkammer ein Druckniveau aufweist, bei dem zum Entfernen des gebildeten Kondensates aus den Sterilisationskammern die beiden Komponenten der Kondensates verdampfen, dass die Unterdruckkammern als Ringkanäle ausgebildet sind, mit denen die Sterilisationskammern über Ventile verbindbar sind und dass jeder Sterilisationskammer ein eigener kleiner Verdampfer zugeordnet ist.

Die Aufgabe wird auch dadurch gelöst, dass die dem ersten Sektor zugeordnete erste Unterdruckkammer ein Druckniveau aufweist, das ein Einströmen eines Wasserdampf und Wasserstoffperoxiddampf enthaltenden Dampfgemisches in die Sterilisationskammern sowie ein kondensierendes Dampfgemisches an den Oberflächen der zu sterilisierenden Behälter ermöglicht, dass die dem zweiten Sektor zugeordnete zweite Unterdruckkammer ein Druckniveau aufweist, bei dem zum Entfernen des gebildeten Kondensates aus den Sterilisationskammern die beiden Komponenten des Kondensates verdampfen, dass die Unterdruckkammern als Ringkanäle ausgebildet sind, mit denen die Sterilisationskammern über Ventile verbindbar sind, dass den Sterilisationskammern ein weiterer Ringkanal zugeordnet ist, der als Verteiler zum Bereitstellen des Dampfgemisches ausgebildet ist und dass der Verteiler als Verdampfer ausgebildet ist.

Die Aufgabe wird auch durch ein Verfahren zum Sterilisieren von zu befüllenden Behältern mit folgenden Schritten gelöst:

Aufnehmen jeweils eines Behälters in eine von mehreren Sterilisationskammern, Antreiben der Sterilisationskammern in einer kontinuierlichen Förderbewegung, so dass die Sterilisationskammern in zeitlichem Abstand wenigstens zwei stationär angeordnete Sektoren durchlaufen, Einstellen unterschiedlicher Druckniveaus in den Sterilisationskammern beim Durchlaufen der wenigstens zwei Sektoren mittels Anschließen der Sterilisationskammern an eine dem jeweiligen Sektor zugeordnete Unterdruckkammer, sowie Erzeugen eines Wasserdampf und Wasserstoffperoxiddampf enthaltenden Dampfgemisches, Einstellen eines ersten Druckniveaus in der dem ersten Sektor zugeordneten ersten Unterdruckkammer, das ein Einströmen des Dampfgemisches in die Sterilisationskammer sowie ein Kondensieren des Dampfgemisches an den Oberflächen der zu sterilisierenden Behälter ermöglicht, und Einstellen eines zweiten Druckniveaus in der dem zweiten Sektor zugeordneten zweiten Unterdruckkammer, das ein Entfernen des gebildeten Kondensats aus den Sterilisationskammern durch Verdampfen der beiden Komponenten des Kondensates ermöglicht.

Im Gegensatz zu der bekannten Vorrichtung, bei welcher die verschiedenen Druckstufen lediglich technisch sinnvolle Zwischenstufen auf dem Wege zum Erreichen eines angestrebten niedrigen Druckniveaus darstellen, sind bei der erfindungsgemäßen Vorrichtung auf Grund eines anderen Sterilisierverfahrens den verschiedenen Druckstufen verschiedene Prozessschritte zugeordnet. Da gemäß der Erfindung das niedrigste Druckniveau etwa eine Zehnerpotenz höher liegt, liegt nicht nur auf Grund des geänderten Sterilisierverfahrens, sondern auch wegen der unterschiedlichen Evakuierungsgrade bei der erfindungsgemäßen Vorrichtung ein höherer Wirkungsgrad vor. Das ganze Verfahren läuft insgesamt schneller ab als beim Stand der Technik.

Beim Sterilisieren unter Verwendung von Wasserstoffperoxid, das immer in wässriger Lösung vorliegt, geschieht die eigentliche Keimabtötung rein chemisch, durch die Einwirkung "aktivierten" Wasserstoffperoxids. Der hierbei gebrauchte Begriff "Aktivieren" ist an sich undefiniert, doch findet erfahrungsgemäß durch geeignete Wärmezufuhr am Wasserstoffperoxid eine chemische und/oder physikalische Veränderung statt, die letztlich die bei der erfindungsgemäßen Vorrichtung praktizierte Keimabtötung bewirkt. Bei der erfindungsgemäßen Vorrichtung erfolgt das "Aktivieren" des Wasserstoffperoxids genau dann, wenn es zum Sterilisieren gebraucht wird, also bei der Kondensation. Das Entfernen der Wasserstoffperoxidreste wird durch bloßes Evakuieren auf einen Druck unterhalb der Siedepunkte von Wasser und Waserstoffperoxid durchgeführt, was in Sekundenschnelle möglich ist und sämtliche Wasserstoffperoxidrückstände sicher entfernt.

Es hat sich erwiesen, dass der ganz überwiegende Teil des Sterilisationseffektes tatsächlich im Augenblick des Auskondensierens eintritt. Unter "Augenblick" ist dabei der Zeitraum zu verstehen, der für das Auskondensieren aus der Gasphase benötigt wird. Abhängig von der technischen Ausgestaltung können dies einige wenige Zehntelsekunden sein, insbesondere bei schlagartiger adiabatischer Expansion, oder aber auch wenige Sekunden bei anderer Ausgestaltung eines hierfür benötigten Verdampfers.

Die Anmelderin vermutet, dass die "Aktivierung" des Wasserstoffperoxids durch die beim Kondensieren frei werdende Verdampfungsenthalpie hervorgerufen wird. Die bei der Kondensation frei werdende Verdampfungsenthalpie liefert die nötige Energie, um ein Wasserstoffperoxydmolekül derart dissoziieren zu können, dass ein Sauerstoffatom frei wird. Vermutlich ist dieser chemisch hoch reaktive atomare Sauerstoff für die keimabtötende Wirkung verantwortlich.

Zur Einflussnahme auf die Sterilisationsleistung stehen als Parameter die Dampftemperatur, der Druck des Dampfes vor dem Einströmen in die Sterilisationskammern sowie der Druck, bei dem die Kondensation erfolgt, zur Verfügung. Von diesen Parametern hat offensichtlich der Druck, bei dem die Kondensation in der jeweiligen Sterilisationskammer erfolgt, den größten Einfluss auf die Sterilisationswirkung im Kondensationsaugenblick. Versuche haben gezeigt, dass der Kondensationsdruck auf jeden Fall unter etwa 500 mbar liegen sollte und dass anzustreben ist, dass sich der Kondensationsdruck dem Wert von etwa 250 bis 150 mbar nähert.

Bezüglich des Unterdruckes beim Entfernen des Kondensates hat sich durch Versuche gezeigt, dass offensichtlich ein Unterdruck von etwas unter 4 mbar erforderlich ist.

Vorteilhaft sind die Unterdruckkammern jeweils als Ringkanäle ausgebildet, mit denen die einzelnen Sterilisationskammern in den betreffenden stationären Sektoren über Ventile verbindbar sind. Auch das sterilisierende Dampfgemisch aus Wasserdampf und Wasserstoffperoxyddampf kann über einen entsprechenden Ringkanal bereitgestellt werden, aus dem das Dampfgemisch über geeignete Ventile in die Sterilisationskammern des entsprechenden Sektors gelangt. Dieser als Verteiler wirkende Ringkanal könnte selbst der Verdampfer sein, in welchen die wässrige Wasserstoffperoxydlösung direkt und kontinuierlich eingespritzt wird. Der Ringkanal könnte aber alternativ auch ein Speicherbehälter für das Dampfgemisch sein, welches in einem separaten Verdampfer er zeugt wird und dann den Speicherbehälter speist. Im letztgenannten Fall findet eine kontinuierliche Dampfzufuhr in den Speicherbehälter statt. Schließlich besteht auch die Möglichkeit, jeder Sterilisationskammer einen eigenen kleinen Verdampfer zuzuordnen.

Nach dem Abziehen des sterilisierenden Kondensats kann die jeweilige Sterilisationskammer durch Öffnen eines entsprechenden Ventils auf Normaldruck geflutet werden, wobei zweckmäßig auch für das flutende Sterilgas ein Ringkanal vorgesehen wird.

Nachfolgend wird ein Ausführungsbeispiel einer erfindungsgemäßen Vorrichtung an Hand einer schematischen Figur erläutert.

Gemäß dieser Figur kann eine Vielzahl von Sterilisationskammern 1 am Umfang eines Rundläufers 2 angeordnet sein, dessen Richtung der Förderbewegung mit A bezeichnet ist. Beispielsweise können 100 derartiger Sterilisationskammern 1 vorhanden sein. Jede dieser Sterilisationskammern 1 nimmt einen zu sterilisierenden Behälter 3 auf. Die Behälter 3 werden beispielsweise über eine nur angedeutete Transporteinrichtung 4 in Zuführrichtung B dem Rundläufer 2 zugeführt. Über einen Einlaufstern 5, der in Pfeilrichtung rotiert, gelangen die Behälter 3 sukzessive in den Bereich unterhalb der Sterilisationskammern 1 und werden in diese eingeschoben. Entsprechend gibt es einen in Pfeilrichtung rotierbaren Auslaufstern 6, über den die sterilisierten, gegebenenfalls auch befüllten und verschlossenen Behälter 7 in Abführrichtung C mit einer Transporteinrichtung 8 wieder abgeleitet werden.

Wenn der Rundläufer 2 beispielsweise mit fünfzig Sterilisationskammern 1 versehen ist und im Takt von 0,2 Sekunden jeweils ein Behälter 3 zugeführt wird, dann beträgt die Umlaufdauer 10 Sekunden. Die Leistung liegt dann in der Größenordnung von 20.000 Behältern pro Stunde.

Der Rundläufer 2 ist um eine Achse 9 angetrieben. Die einzelnen Sterilisationskammern 1 durchlaufen so genannte Sektoren, die dem Rundläufer 2 stationär zugeordnet sind und durch kleine Buchstaben a bis g sowie einen Doppelpfeil gekennzeichnet sind. In jedem dieser Sektoren a bis g wird ein ganz bestimmter Prozessschritt durchgeführt, wobei die einzelnen Sterilisationskammern 1 infolge der Drehung des Rundläufers 2 nacheinander die einzelnen Sektoren durchlaufen.

In den einzelnen Sektoren a bis g werden die folgenden Prozessschritte durchgeführt:
Sektor a: Einbringen der Behälter 3 in die Sterilisationskammern 1 sowie Verschließen der Sterilisationskammern 1.
Sektor b: Evakuieren der Sterilisationskammern 1 auf den Dampfeinlassdruck, also auf einen Druck, der etwas kleiner als etwa 400 mbar ist, vorzugsweise auf einen Druck von etwa 150 mbar.
Sektor c: Kurzzeitiges Einlassen des aus Wasserdampf und Wasserstoffperoxyddampf bestehenden Dampfgemisches.
Sektor d: Abwarten der Kondensation, bei welcher die eigentliche Sterilisation erfolgt.
Sektor e: Abziehen des Kondensates durch Evakuieren der Sterilisationskammern 1 unter den Dampfdruck, vorzugsweise auf einen Unterdruck von etwa 4 mbar.
Sektor f: Fluten mit Sterilluft oder beispielsweise mit sterilem Stickstoff.
Sektor g: Öffnen der Sterilisationskammern 1 und Freigeben der Behälter 3 sowie das Ausziehen der Behälter 3 aus der geöffneten Sterilisationskammer 1.

Je nach Maschinenkonzept beträgt der Gesamtaufwand für die genannten Prozessschritte etwa sechs bis 10 Sekunden.

Das Evakuieren im Sektor b sowie das Evakuieren im Sektor e kann jeweils durch einfaches Öffnen derjenigen zugeordneten Ventile erfolgen, welche die Verbindung der Sterilisationskammern 1 zu als Ringkanäle 10 und 11 ausgebildeten Unterdruckkammern herstellen. Das sterilisierende Dampfgemisch kann in einem ebenfalls als Ringkanal ausgebildeten Verteiler 12 bereitgestellt werden. Aus diesem gelangt das Dampfgemisch über geeignete Ventile in die Sterilisationskammern 1 (Sektor c). Die Dampfmenge wird bei vorgegebenem Druck im Verteiler 12 über die Öffnungsdauer gesteuert.

Der als Ringkanal ausgebildete Verteiler 12 kann, wie in der schematischen Figur dargestellt, selbst der Verdampfer sein, in welchen die Wasserstoffperoxidlösung in bereits erläuterter Weise direkt und kontinuierlich eingespritzt wird.

Durch Öffnen eines entsprechenden Ventils werden schließlich die Sterilisationskammern 1 auf Normaldruck geflutet (Sektor f). Auch für dieses Sterilgas ist ein Ringkanal 13 vorgesehen. Es könnte gebenenfalls zweckmäßig sein, auf Überdruck zu fluten, damit ein Eindringen des umgebenden Gasgemisches in die Behälter 3 verhindert oder vermindert wird. Hierzu kann dann zweckmäßig Stickstoff verwendet werden, um den atmosphärischen Sauerstoff abzuhalten. Gegebenenfalls könnte es wünschenswert sein, alternativ bei geringem Unterdruck zu fluten, um Zeit zu sparen.

Ein ortsfester, neben dem Rundläufer 2 stehender Pumpstand 14 erzeugt im Ringkanal 10 den Dampfeinlassdruck. Da 150 mbar ein ausreichend niedriges Druckniveau ist, wird hierfür vorteilhaft eine Drehschieberpumpe eingesetzt.

Das Abziehen des Kondensates übernimmt ein Pumpstand 15, der im Ringkanal 11 einen Druck aufrechtzuerhalten hat, bei dem die Komponenten des Kondensates, nämlich Wasser und Wasserstoffperoxyd, verdampfen. Dieses Druckniveau beträgt etwa 4 mbar. Auf Grund dieses bereits relativ niedrigen Wertes besteht der Pumpstand zweckmäßigerweise aus einer Wälzkolben-/Drehschieberkombination. Es ist deshalb zweckmäßig, die Wälzkolbenpumpe des Pumpstandes 15 mit dem Rundläufer 2 mit rotieren zu lassen, denn das abgepumpte Gasgemisch müsste anderenfalls durch die sich drehende Nabe des Rundläufers 2 hindurch abgesaugt werden.

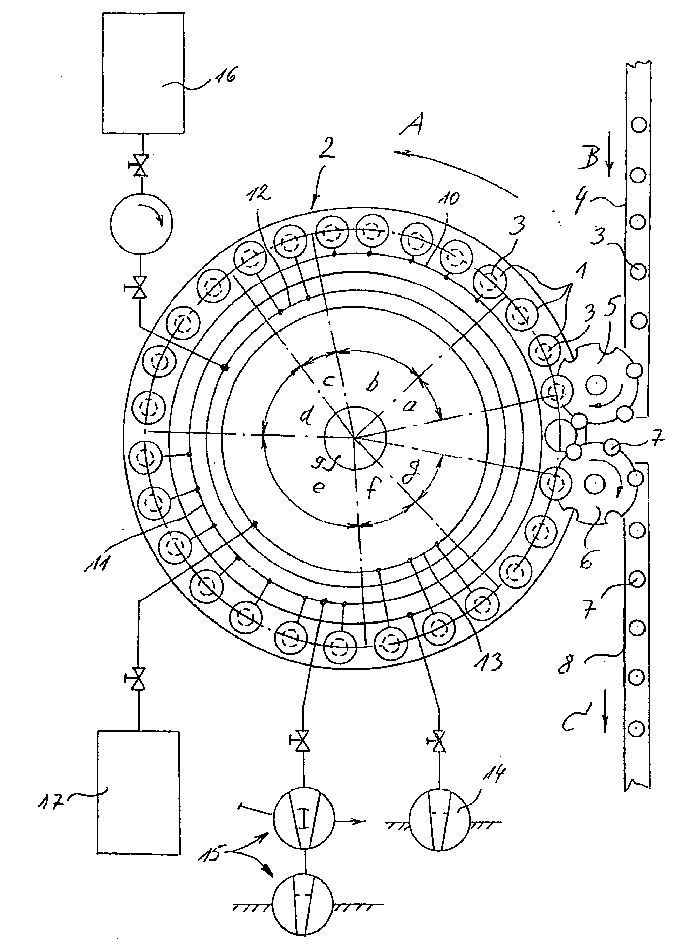

## Patentansprüche

1. Vorrichtung zum Sterilisieren von zu befüllenden Behältern, mit einer Vielzahl von mit einer kontinuierlichen oder getakteten Förderbewegung antreibbaren, jeweils einen Behälter aufnehmenden Sterilisationskammern, die in zeitlichem Abstand wenigstens zwei stationär angeordnete Sektoren durchlaufen, denen jeweils eine Unterdruckkammer unterschiedlichen Druckniveaus zugeordnet ist, an welche die Sterilisationskammern anschließbar sind, wobei die Unterdruckkammern als Ringkanäle (10, 11) ausgebildet sind, mit denen die Sterilisationskammern (1) über Ventile verbindbar sind, **dadurch gekennzeichnet, dass** jeder Sterilisationskammer (1) ein eigener kleiner Verdampfer zum Erzeugen eines Wasserdampf und Wasserstoffperoxyddampf enthaltenden Dampfgemisches zugeordnet ist, dass die dem ersten Sektor (b) zugeordnete erste Unterdruckkammer (10) ein Druckniveau aufweist, das ein Einströmen des Wasserdampf und Wasserstoffperoxyddampf enthaltenden Dampfgemisches in die Sterilisationskammern (1) sowie ein Kondensieren des Dampfgemisches an den Oberflächen der zu sterilisierenden Behälter (3) ermöglicht, und dass die dem zweiten Sektor (e) zugeordnete zweite Unterdruckkammer (11) ein Druckniveau aufweist, bei dem zum Entfernen des gebildeten Kondensates aus den Sterilisationskammern (1) die beiden Komponenten des Kondensates verdampfen.

2. Vorrichtung zum Sterilisieren von zu befüllenden Behältern, mit einer Vielzahl von mit einer kontinuierlichen oder getakteten Förderbewegung antreibbaren, jeweils einen Behälter aufnehmenden Sterilisationskammern, die in zeitlichem Abstand wenigstens zwei stationär angeordnete Sektoren durchlaufen, denen jeweils eine Unterdruckkammer unterschiedlichen Druckniveaus zugeordnet ist, an welche die Sterilisationskammern anschließbar sind, wobei die Unterdruckkammern als Ringkanäle (10, 11) ausgebildet sind, mit denen die Sterilisationskammern (1) über Ventile verbindbar sind, **dadurch gekennzeichnet, dass** wenigstens ein Verdampfer zum Erzeugen eines Wasserdampf und Wasserstoffperoxyddampf enthaltenden Dampfgemisches vorgesehen ist, dass die dem ersten Sektor (b) zugeordnete erste Unterdruckkammer (10) ein Druckniveau aufweist, das ein Einströmen des Wasserdampf und Wasserstoffperoxyddampf enthaltenden Dampfgemisches in die Sterilisationskammern (1) sowie ein Kondensieren des Dampfgemisches an den Oberflächen der zu sterilisierenden Behälter (3) ermöglicht, und dass die dem zweiten Sektor (e) zugeordnete zweite Unterdruckkammer (11) ein Druckniveau aufweist, bei dem zum Entfernen des gebildeten Kondensates aus den Sterilisationskammern (1) die beiden Komponenten des Kondensates verdampfen, und dass den Sterilisationskammern (1) ein weiterer Ringkanal zugeordnet ist, der als Verteiler (12) zum Bereitstellen des Dampfgemisches ausgebildet ist, und dass der Verteiler (12) als Verdampfer ausgebildet ist.

3. Vorrichtung nach Anspruch 2, **dadurch gekennzeichnet, dass** in den Verteiler (12) eine aus Wasser und Wasserstoffperoxyd bestehende Lösung einspritzbar ist.

4. Verfahren zum Sterilisieren von zu befüllenden Behältern mit einer Vorrichtung gemäß Anspruch 1 oder 2, wobei das Verfahren die Schritte aufweist:
Aufnehmen jeweils eines Behälters in eine von mehreren Sterilisationskammern (1),
Antreiben der Sterilisationskammern (1) in einer kontinuierlichen Förderbewegung, so dass die Sterilisationskammern (1) in zeitlichem Abstand wenigstens zwei stationär angeordnete Sektoren (b, e) durchlaufen,
Einstellen unterschiedlicher Druckniveaus in den Sterilisationskammern (1) beim Durchlaufen der wenigstens zwei Sektoren (b, e) mittels Anschließen der Sterilisationskammern (1) an eine dem jeweiligen Sektor (b, e) zugeordnete Unterdruckkammer (10,11)
**gekennzeichnet durch**
Erzeugen eines Wasserdampf und Wasserstoffperoxyddampf enthaltenen Dampfgemisches,
Einstellen eines ersten Druckniveaus in der dem ersten Sektor (b) zugeordneten ersten Unterdruckkammer (10), das ein Einströmen des Dampfgemisches in die Sterilisationskammer (1) sowie ein Kondensieren des Dampfgemisches an den Oberflächen der zu sterilisierenden Behälter (3) ermöglicht, und
Einstellen eines zweiten Druckniveaus in der dem zweiten Sektor (e) zugeordneten zweiten Unterdruckkammer (11), das ein Entfernen des gebildeten Kondensats aus den Sterilisationskammern (1) **durch** Verdampfen der beiden Komponenten des Kondensates ermöglicht.

## Claims

1. Device for sterilising receptacles to be filled, with multiple sterilisation chambers that each accept one receptacle at a time and which can be powered by a continuous or a timed conveying motion, whereby the sterilisation chambers pass at different time intervals through at least two stationary sectors which are each allocated a vacuum chamber of a differing pressure level to which the sterilisation chambers can be connected, whereby the vacuum chambers are designed as ring channels (10, 11) with which the sterilisation chambers (1) can be connected via valves, **characterised in that** each sterilisation chamber (1) is allocated its own small vaporiser for generating a vapour mixture containing water vapour and hydrogen peroxide vapour, and **characterised in that** the first vacuum chamber (10) allocated to the first sector (b) features a pressure level that enables the vapour mixture containing water vapour and hydrogen peroxide vapour to flow into the sterilisation chambers (1) and enables the vapour mixture to condense on the surfaces of the receptacles (3) to be sterilised, and **characterised in that** the second vacuum chamber (11) allocated to the second sector (e) features a pressure level at which the two elements of the condensate vaporise and thereby remove the condensate that has formed from the sterilisation chambers (1).

2. Device for sterilising receptacles to be filled, with multiple sterilisation chambers that each accept one receptacle at a time and which can be powered by a continuous or a timed conveying motion, whereby the sterilisation chambers pass at different time intervals through at least two stationary sectors which are each allocated a vacuum chamber of a differing pressure level to which the sterilisation chambers can be connected, whereby the vacuum chambers are designed as ring channels (10, 11) with which the sterilisation chambers (1) can be connected via valves, **characterised in that** at least one vaporiser is provided to generate a vapour mixture containing water vapour and hydrogen peroxide vapour, that the first vacuum chamber (10) allocated to the first sector (b) features a pressure level that enables the vapour mixture containing water vapour and hydrogen peroxide vapour to flow into the sterilisation chambers (1) and enables the vapour mixture to condense on the surfaces of the receptacles (3) to be sterilised, and that the second vacuum chamber (11) allocated to the second sector (e) features a pressure level at which the two elements of the condensate vaporise and thereby remove the condensate that has formed from the sterilisation chambers (1), and that the sterilisation chambers (1) are allocated an additional ring channel that is designed as a distributor (12) for supplying the vapour mixture, and that the distributor (12) is designed as a vaporiser.

3. Device according to Claim 2, **characterised in that** a solution composed of water and hydrogen peroxide can be injected into the distributor (12).

4. Process for sterilising receptacles to be filled, using a device according to Claim 1 or 2, whereby the process features the following steps:
acceptance of one receptacle at a time in one of several sterilisation chambers (1),
powering of the sterilisation chambers (1) via a continuous conveying motion so that the sterilisation chambers (1) pass at different time intervals through at least two stationary sectors (b, e),
adjustment of differing pressure levels in the sterilisation chambers (1) during traversal of the minimum two sectors (b, e) via connection of the sterilisation chambers (1) to a vacuum chamber (10, 11) allocated to one of the respective sectors (b, e),
**characterised by**
generation of a vapour mixture containing water vapour and hydrogen peroxide vapour,
adjustment of the first pressure level in the first vacuum chamber (10) allocated to the first sector (b) such that the vapour mixture can flow into the sterilisation chambers (1) and can condense on the surfaces of the receptacles (3) to be filled, and
adjustment of the second pressure level in the second vacuum chamber (11) allocated to the second sector (e) such that the two elements of the condensate can vaporise to remove the condensate that has formed from the sterilisation chambers.

## Revendications

1. Dispositif destiné à stériliser des récipients à remplir, avec un grand nombre de chambres de stérilisation recevant respectivement un récipient, pouvant être entraînées avec un mouvement d'alimentation continu ou cadencé, lesquelles chambres traversent dans un laps de temps au moins deux secteurs disposés de manière fixe, auxquels est respectivement attribué une chambre de dépression à niveaux de pression différents, auxquelles peuvent être raccordées les chambres de stérilisation, les chambres de dépression étant configurées comme des canaux annulaires (10, 11) auxquels peuvent être reliées les chambres de stérilisation (1) au moyen de soupapes, **caractérisé en ce qu'**à chaque chambre de stérilisation (1) est attribué un petit évaporateur individuel destiné à produire un mélange vapeur contenant de la vapeur d'eau et de la vapeur de peroxyde d'hydrogène, **en ce que** la première chambre de dépression (10) attribuée au premier secteur (b) présente un niveau de pression qui permet d'introduire le mélange vapeur contenant de la vapeur d'eau et de la vapeur de peroxyde d'hydrogène dans les chambres de stérilisation (1) ainsi que de condenser le mélange vapeur sur les surfaces des récipients (3) à stériliser, et **en ce que** la deuxième chambre de dépression (11) attribuée au deuxième secteur (e) présente un niveau de pression dans lequel les deux composants du condensat s'évaporent pour éliminer des chambres de stérilisation (1) le condensat formé.

2. Dispositif destiné à stériliser des récipients à remplir, avec un grand nombre de chambres de stérilisation recevant respectivement un récipient, pouvant être entraînées avec un mouvement d'alimentation continu ou cadencé, lesquelles chambres traversent dans un laps de temps au moins deux secteurs disposés de manière fixe, auxquels est respectivement attribuée une chambre de dépression à niveaux de pression différents, auxquelles peuvent être raccordées les chambres de stérilisation, les chambres de dépression étant configurées comme des canaux annulaires (10, 11) auxquels peuvent être reliées les chambres de stérilisation (1) au moyen de soupapes, **caractérisé en ce qu'**au moins un évaporateur destiné à produire un mélange vapeur contenant de la vapeur d'eau et de la vapeur de peroxyde d'hydrogène est prévu, **en ce que** la première chambre de dépression (10) attribuée au premier secteur (b) présente un niveau de pression qui permet d'introduire le mélange vapeur contenant de la vapeur d'eau et de la vapeur de peroxyde d'hydrogène dans les chambres de stérilisation (1) ainsi que de condenser le mélange vapeur sur les surfaces des récipients (3) à stériliser, et **en ce que** la deuxième chambre de dépression (11) attribuée au deuxième secteur (e) présente un niveau de pression dans lequel les deux composants du condensat s'évaporent pour éliminer des chambres de stérilisation (1) le condensat formé et **en ce qu'**un autre canal annulaire est attribué aux chambres de stérilisation (1), lequel est configuré comme un distributeur (12) pour mettre à disposition le mélange vapeur et **en ce que** le distributeur (12) est configuré comme un évaporateur.

3. Dispositif selon la revendication 2, **caractérisé en ce qu'**une solution se composant d'eau et de peroxyde d'hydrogène est injectable dans le distributeur (12).

4. Procédé de stérilisation de récipients à remplir avec un dispositif selon les revendications 1 ou 2, le procédé comportant les étapes suivantes :
réception chaque fois d'un récipient dans l'une de nombreuses chambres de stérilisation (1),
entraînement des chambres de stérilisation (1) dans un mouvement de transport continu, de manière à ce que les chambres de stérilisation (1) traversent dans un laps de temps au moins deux secteurs (b, e) disposés de manière fixe,
réglage de différents niveaux de pression dans les chambres de stérilisation (1) lors de la traversée des au moins deux secteurs (b, e) par raccordement des chambres de stérilisation (1) à l'une des chambres de dépression (10, 11) attribuées à chaque secteur respectif (b, e),
**caractérisé par**
la production d'un mélange vapeur contenant de la vapeur d'eau et de la vapeur de peroxyde d'hydrogène.
réglage d'un premier niveau de pression dans la première chambre de dépression (10) attribuée au premier secteur (b), qui permet une introduction du mélange vapeur dans la chambre de stérilisation (1) ainsi qu'une condensation du mélange vapeur sur les surfaces des récipients (3) à stériliser et
réglage d'un deuxième niveau de pression dans la deuxième chambre de dépression (11) attribuée au deuxième secteur (e), qui permet d'éliminer par évaporation des deux composants du condensat le condensat formé des chambres de stérilisation (1).
